# EUROPEAN PATENT APPLICATION

(11) **EP 4 810 016 A1**
(43) Date of publication of application: **23.09.2026**
(21) Application number: 25165468.7
(22) Date of filing: 21.03.2025
(51) Int. Cl.: A61K 9/00, A61K 31/495, A61P 25/18, A61P 25/24

(54) **ORAL THIN FILM FOR THE ADMINISTRATION OF CARIPRAZINE**

(71) Applicant: LTS LOHMANN Therapie-Systeme AG, 56626 Andernach (DE)
(72) Inventor: HAMMES, Dr. Florian, 56626 Andernach (DE); LINN, Dr. Michael, 55596 Waldböckelheim (DE); BAUER, Dr. Marius, 56626 Andernach (DE); KLEUDGEN, Tobias, 56729 Ettringen (DE)
(74) Representative: Meissner Bolte Partnerschaft mbB

(57) **Abstract**

The present invention concerns a therapeutic system in the form of an oral thin film for the administration of cariprazine, which comprises a matrix layer. The matric layer is mainly formed from cariprazine or a pharmaceutically acceptable salt, tautomer or stereoisomer thereof and a film forming agent comprising a water-soluble polymer, which is selected from pullulan and polyvinyl alcohol and where the pullulan and/or polyvinyl alcohol accounts for 57 wt.-% or more of the matrix layer. The respective oral thin films provide for simplified administration which maintains bioequivalence to approved oral dosage forms of cariprazine. The present invention is further concerned with methods for the production of respective therapeutic systems and respective oral thin films for use as a medicament and for the treatment of mental diseases.

## Description

The present invention concerns a therapeutic system in the form of an oral thin film for the administration of cariprazine, which comprises a matrix layer. The matrix layer is mainly formed from cariprazine or a pharmaceutically acceptable salt, tautomer or stereoisomer thereof and a film forming agent comprising a water-soluble polymer, which is selected from pullulan and polyvinyl alcohol and where the pullulan and/or polyvinyl alcohol accounts for 57 wt.-% or more of the matrix layer. The present invention is further concerned with methods for the production of respective therapeutic systems and respective oral thin films for use as a medicament and for the treatment of mental diseases.

### State of the art

Cariprazine is a dopamine D3-preferring D3/D2 receptor partial agonist, which was first disclosed in WO 2005/012266 A1/US 7737142 B2 along with its respective pharmaceutically acceptable salts. This document also discloses pharmaceutical compositions containing hydrochloride or other pharmaceutically acceptable salts of cariprazine and their use for therapy and/or prevention of pathological conditions which require the modulation of dopamine receptors. Such conditions include i.a. psychoses (e.g. schizophrenia, schizo-affective disorders, etc.), drug abuse (e.g. alcohol, cocaine, nicotine, opioids, etc.), cognitive impairment accompanying schizophrenia (including positive symptoms, such as delusions and hallucinations, and negative symptoms, such as lack of drive and social withdrawal, and cognitive symptoms, such as problems with attention and memory), mild-to-moderate cognitive deficits, dementia, psychotic states associated with dementia, eating disorders (e.g. bulimia nervosa, etc.), attention deficit disorders, hyperactivity disorders in children, psychotic depression, mania, paranoid and delusional disorders, dyskinetic disorders (e.g. Parkinson's disease, neuroleptic induced parkinsonism, tardive dyskinesias) anxiety, sexual dysfunction, sleep disorders, emesis, aggression, and autism. Cariprazine has in the meantime been approved by the FDA and EMA as is available in capsule form, and is available as Vraylar or Reagila.

The available preparations of caripraizine are mainly cariprazine hydrochloride immediate release (IR) forms. In this respect, in addition to the disclosure in WO 2005/012266 A1, WO 2010/009309 A1 discloses stable and bioavailable immediate release pharmaceutical compositions of the drug. WO 2009/104739 A1 discloses a solid preparation for oral administration of cariprazine hydrochloride as an immediate release tablet dosage form. Further immediate release dosage forms of cariprazine hydrochloride are described in EP 16165247 A1, which discloses granules, fine granules or powders having superior properties. In addition, some delayed cariprazine dosage forms have been described in e.g. WO 2018229641 A1, where the cariprazine is formulated as a tablet with a release modifying agent.

In addition to administration forms, where the drug is taken orally, but absorbed only in the intestinal tract, there have been attempts to formulate cariprazine for direct oral absorption. In this respect, WO 2022247883 A1 describes orally dissolving films, where the cariprazine is formulated with a specific low particle size or as an inclusion compound with cyclodextrine to improve uptake of the drug via the oral mucosa.

A problem with attempts to administer cariprazine via uptake in the oral cavity is that the drug has poor oral absorption, so that there is partial uptake of the drug via the oral mucosa and in the gastrointestinal tract. Since this changes the bioavailability profile of the drug and up to now drug approval has only been obtained for conventional oral dosage forms with drug uptake in the gastrointestinal tract, this approach faces the difficulty of the necessity of a new drug approval.

On the other hand, a dosage form as an orally dissolving thin film has the advantage of facilitated administration and improved administration compliance. This is especially important as persons suffering from mental diseases are often not cooperative towards treatment with drugs of may have swallowing difficulties. Accordingly, there is a need for a means of administration of cariprazine which provides facilitated administration and administration compliance and has bioequivalence to Vraylar.

The present application addresses this need. In particular, it is an object of the present invention to provide an improved oral thin film for the administration of cariprazine. Further, it is a further object of the present invention to provide an oral thin film for the administration of cariprazine which is characterized by fast dissolution time so to assure absorption of the drug predominantly in the gastrointestinal tract. A further object of the present invention is to provide an alternative administration form to conventional cariprazine dosage forms, which is bioequivalenet to Varylar.

### Detailed description of the invention

In the investigation which are underlying the present invention, it has been found that the formulation of cariprazine as a fast dissolving oral thin film, which is based on polyvinyl alcohol or pullulan as film forming an active agent binding polymer, provides for fast dissolution when a respective film is put into the oral cavity and for fast release of the drug. On the other hand, the polyvinyl alcohol or pullulan do not facilitate uptake of the drug via the oral mucosa, so that the drug is not absorbed but swallowed by the patient, to which the film has been administered. In this manner, it is ensured that the drug is mainly absorbed in the gastrointestinal tract and the administration form has bioequivalence to approved forms of cariprazine (i.e. Vraylar).

Accordingly, in a first aspect, the present invention is directed to a therapeutic system in the form of an oral thin film for the administration of cariprazine, which comprises a matrix layer, and wherein the matrix layer comprises:
- cariprazine or a pharmaceutically acceptable salt, tautomer or stereoisomer thereof; and
- film forming agent comprising a water-soluble polymer, which is selected from pullulan and polyvinyl alcohol,
wherein pullulan and/or polyvinyl alcohol account for 57 wt.-% or more of the matrix layer.

In the respective matrix layer, the water-soluble polymer provides for the predominant part of the composition, which ensures that if the film is taken into the oral cavity and comes in contact with saliva, the film rapidly dissolves and releases the cariprazine active ingredient.

When in the following reference is made to the "oral thin film of the invention" or the "oral thin film according to the present invention", this is intended to denote the "therapeutic system" of the invention.

As noted above, the invention is intended to deliver and release the active ingredient in the oral cavity of a patient, but it is not intended that there is substantial absorption of the active ingredient in the oral cavity. Accordingly, it is preferred in the context of the invention that the cariprazine is not provided in a form which facilitates oral uptake or absorption thereof. In particular, it is preferred that the cariprazine is not provided in complexed form, as in e.g. a complex with cyclodextrines, as such formulation would significantly improve the dissolution of cariprazine and thereby provide for improved oral absorption. In addition, it is preferred that the cariprazine is not provided as a powder with a very fine particle size, as such processing is also prone to enhance the oral absorption and uptake of active ingredients. The carpraizine is thus preferably not in nanoparticulate form and more preferably has a D90 particle size of > 30 µm, more preferably ≥ 40 µm and even more preferably ≥ 50 µmv. As on the other hand, there should also not be particles with a size which is larger than the thickness of the film prepared with the cariprazine, the D90 particle size of the cariprazine is preferably equal to or less than 100 µm, more preferably equal to or less than 90 µm and even more preferably equal to or less than 80 µm. In a particularly preferred embodiment, the D90 particle size of the cariprazine is in the range from 50 to 80 µm.

In the investigations, which are underlying the present invention, it has been found that some water-soluble polymers have a favorable influence on oral absorption of the cariprazine in the oral cavity. Water soluble polymers, for which such effect has been observed, are e.g. hydroxypropylmethylcellulose (HMPC) and xanthan gum. As the intention of the invention is to prevent such uptake, it is therefore preferred that the inventive film does not comprise either of these water-soluble polymers.

The content of the cariprazine in the oral thin film of the invention will usually be such that on the one hand the content of water-soluble polymer is not excessive (for cost considerations), and on the other hand it is ensured that the oral thin film is sufficiently stable for handling by the patient, and that sufficient amounts of auxiliary additive can be accommodated in the film. Thus, suitably, the film comprises 2 to 20 wt.-% and preferably 5 to 15 wt.-% of cariprazine.

The cariprazine is further preferably used in the form of a pharmaceutically acceptable salt, as in this form is can conveniently be prepared with sufficient purity for pharmaceutical application. Suitable pharmaceutically acceptable salts include i.a. salts with inorganic acids such as hydrochloric acid, sulfuric acid, nitric acid or phosphoric acid, salts with monovalent organic acids such as formic acid, acetic acid, propionic acid, and different butyric acids, valeric acids or capric acids, salts with bivalent organic acids such as oxalic acid, malonic acid, maleic acid, fumaric acid or salts with other organic acids such as hydroxy acids including citric acid and tartaric acid, or aromatic carboxylic acids including benzoic acid or salicylic acid, as well as aliphatic and aromatic sulfonic acids, e.g. methanesulfonic acid, naphtalenesulfonic acid or p-toluenesulfonic acid succinic acid. An especially valuable group of the acid addition salts are salts in which the acid component itself is physiologically acceptable and does not have therapeutical effect in the applied dose or it does not have unfavourable influence on the effect or uptake of the active ingredient. Most preferably the cariparzine is in the form of cariparzine hydrochloride, which is the form having market approval for therapy.

In addition to the pullulan and/or polyvinyl alcohol, the matrix layer may comprise further water-soluble polymer. However, as pullulan and polyvinyl alcohol have been found to not influence the uptake of cariprazine in a manner that there is subatntial uptake in the oral cavity, it is preferred in the invention that the combined amount of pullulan and/or polyvinyl alcohol on the total weight of water-soluble polymers in the matrix layer is at least 80 wt.-%, more preferably at least 90 wt.-% and even more preferably at least 95 wt.-%.

In the investigations, which are underlying the present invention, it has further been found that films, which are formulated with polyvinyl alcohol, on administration provide the lowest drug uptake via the mucosa, so that in a preferred embodiment, the matrix layer is based on polyvinyl alcohol as the water-soluble polymer. Particularly suitable forms of polyvinyl alcohol have a molecular weight Mw (as determined by GPC with appropriate standard) in the range from 20,000 to 70,000 g/mol and more preferably 25,000 to 35,000 g/mol. Such forms of polyvinyl alcohol provide for very fast dissolution of the film in an aqueous environment.

It is furthermore preferred that the polyvinyl alcohol has a degree of hydrolysis in the range from 80 % to 95% and more preferably 84 % to 92 %, and/or a viscosity as a 4% aqueous solution at 20°C according to DIN 53015 of 1 to 10 mPa.s and preferably 2 to 7 mPa.s.

As noted above, the content of polyvinyl alcohol and pullulan as water soluble polymers in the oral thin films of the invention is at least 57% by weight of the matrix layer. The upper limit of the content of these polymers is mainly dependent on the amount of cariparizine, which is incorporated into the film, as well as further auxiliary agents, which may be comprised therein. In this respect, it is preferred that the upper limit content of the water-soluble polymer is 80 wt.-% or less, more preferably 75 wt.-% or less, even more preferably 70 wt.-% or less and even more preferably 65 wt.-% or less.

In oral thin films, which are formulated with polyvinyl alcohol as water soluble polymer, the weight ratio, in which the cariprazine or a pharmaceutically acceptable salt, tautomer or stereoisomer thereof and polyvinyl alcohol are present therein is depending on the amount of cariprazine, which is incorporated into the therapeutic system. Suitably, this ratio is in the range from 1:3 to 1:6 and preferably 1:3.5 to 1:5 (i.e. there is a relevant excess of the polyvinyl alcohol).

Whereas the inventive oral thin film can be produced from only a combination of cariprazine as the active agent and water-soluble polymer as a film forming agent and carrier of the active agent, the oral thin film will normally comprise additional auxiliary additives to e.g. improve one or more of mechanical or administration related properties of the film.

An important auxiliary agent of this type is e.g. a plasticizer, which improves flexibility and toughness of the film. Accordingly, it is preferred that the matrix layer in the inventive oral thin film comprises one or more plasticizers. As exemplary plasticizers, which can be used in oral thin films according to the invention, polyethylene glycol, glycerol, propylene glycol, silicone oil, simethicone, polypropylene glycol and hexylene glycol can be mentioned. Among these, glycerol is particularly preferred.

The content of plasticizer in the film is usually adjusted such that the desired amount of plasticization is obtained, which the skilled practitioner can determine depending on his respective needs. Particularly suitably, the content of the one or more plasticizers in the inventive oral thin film is adjusted in the range from 2 to 15 wt.-% and more particularly 2.5 to 10 wt.-%, based on the weight of the matrix layer.

Another auxiliary additive, which can suitably be used in the oral thin films according to the invention, is a humectant. Accordingly, for the inventive oral thin film is it preferred that the matrix layer thereof also comprises one or more humectants. Possible humectants, which can be used in the films include polyglycols such as propylene glycol, butylene glycol or hexylene glycol, α-hydroxy acids such as lactic acid, glyceryl triacetate, polymeric polyols such as polydextrose and sugar alcohols such as sorbitol, xylitol and maltitol. Such humectants are preferably incorporated in amounts from 4 to 15 wt.-% and more preferably 6 to 12 wt.-%, based on the total weight of the matrix layer.

In addition to the above-mentioned additives, the oral thin film of the invention may further comprise one or more additives selected from the group comprising fatty acids, sweeteners, flavoring agents, colorants, taste masking agents, solubilizers, disintegrants, dispersing agents, emulsifiers, antioxidants, preservatives, stabilizers, and pH regulators.

Suitable sweetener for use in the oral thin films of the invention may be selected from one or more of aspartame, sucralose, fructose, sucrose, stevioside, glycyrrhizin, essence, fragrance, saccharin and saccharin Na. Suitable emulsifiers include polysorbates such as polysorbate 80 (commercially available as Tween^{®} 80). A suitable dispersing agent is sorbitan sesquioleate, which is commercially available e.g. as Span 83 from Croda.

Disintegrants suitable for use in the oral thin films of the invention include low-substituted hydroxypropyl cellulose, crospovidone, croscarmellose sodium, croscarmellose sodium starch, starch (e.g. as rice starch), microcrystalline cellulose, or pregelatin. Saliva stimulating agents can be selected from one or more of citric acid, tartaric acid, malic acid and mannitol. Suitable colorants can be selected from one or more of titanium dioxide, pigments and the like.

For the purpose of optimization, the above noted auxiliary additives are usually incorporated in limited amounts, where an amount of the combined additives is preferably in the range of 5 to 15 wt.-% and more preferably in the range from 8 to 14 wt.-%, based on the total weight of the matrix layer. In this amount, possible contents of plasticizers and humectants and not accounted for.

It should be noted that in pharmaceutical formulations, the formulation components are categorized according to their physicochemical and physiological properties, and in accordance with their function. This means in particular that a substance or a compound falling into one category is not excluded from falling into another category of formulation component. Some substances may e.g. be a typical softener but at the same time act as a permeation enhancer. The skilled person is able to determine based on his general knowledge in which category or categories of formulation component a certain substance or compound belongs to. In the following, details on the excipients and additives are provided which are, however, not to be understood as being exclusive. Other substances not explicitly listed in the present description may be as well used in accordance with the present invention, and substances and/or compounds explicitly listed for one category of formulation component are not excluded from being used as another formulation component in the sense of the present invention.

The oral thin film of the invention is formulated for rapid dissolution in the oral cavity, which can conveniently be achieved when the film has a thickness of equal to or less than 200 µm. On the other hand, if the film become very thin, such as less than 20 µm, the handling of the film becomes more difficult and the stability of the film may also be impaired. Accordingly, it is preferred that the thickness of the film is adjusted in the range of 20 to 150 µm to provide a good compromise of stability and fast dissolution. Particularly preferably, the thickness of the film is in a range from 50 to 120 µm.

In part dependent on the film thickness is the area weight of the films, which in compact films relates to the film thickness via the density. In the invention, an area weight of the film in the range from 20 to 180 g/m² is preferred and an area weight of 50 to 140 g/m² is particularly preferred.

The oral thin film in addition preferably has a size, where it can be inserted into the oral cavity without having to deform or fold the film. To this end, the film is preferably provided with a size of 3 to 10 cm² and more preferably 4 to 6 cm².

For the oral thin film of the invention, the content of cariprazine relative to the size of the film will preferably be in a range from 0.3 to 3.0 mg/cm² and more preferably 0.5 to 2.0 mg/cm². With this amount, pharmaceutically acceptable dosages of cariprazine can be conveniently incorporated in an oral thin film dosage form with the dimensions as noted above.

For the therapeutic system of the invention, as it aims at the provision of cariprazine for main uptake and absorption via the gastrointestinal tract, it is not necessary that the cariprazine is specifically formulated to promote absorption via the oral mucosa. To this end, forms, where the cariprazine is not dissolved in the waters soluble polymer matrix are preferable. Accordingly, it is preferred that the cariprazine or a pharmaceutically acceptable salt, tautomer or stereoisomer thereof in the oral thin film of the invention in dispersed or in microparticulate form in the matrix layer.

Whereas it is possible, that the oral thin film of the invention can be constituted from more than one layer, such additional layers usually increase the complexity of the production of the film. As in addition, the film of the invention is mainly designed for fast dissolution in the oral cavity, it is preferred that the inventive film is a monolayer film. The film can be formulated as a compact film or as a film having voids or bubbles therein to provide a solidified foam film. As the cariprazine, which is used in the film, is suitably used as a comparatively coarse powder (where stable and uniform foam film preparation is difficult), a compact film form is preferred.

As has been noted above, the oral thin films of the invention are mainly designed for fast dissolution and release of the cariprazine into the oral cavity. To this end, it is thus preferred that the oral thin film has a dissolution/disintegration time in the oral cavity of from 8 to 60 sec and more preferably from 10 to 30 sec. Alternatively, or in addition thereto, it is preferred that the oral thin film of the invention is formulated as a flash release film.

In a particularly preferred embodiment, the oral thin film of the present invention comprises the following ingredients:
- 2 to 20 wt.-%, preferably 5 to 15 wt.-% and particularly preferably about 14.5 wt.-% of cariprazine hydrochloride, preferably as uncomplexed cariprazine;
- 57 to 65 wt.-% preferably 57 to 62 wt.-% of polyvinyl alcohol, preferably with a molecular weight Mw of 20,000 to 40,000 g/mol and in particular about 31,000 g/mol;
- 9.0 wt.-% of a humectant, preferably in the form of sorbitol;
- 3 to 9 wt.-% of a plasticizer, preferably in the form of glycerol;
- 5.0 to 6.0 wt.-% of a disintegrant, preferably in the form of rice starch;
- 3.0 wt.-% of sweetener, preferably selected from sucralose and xylitol;
- 1.0 wt.-% of an emulsifier, preferably in the form of polysorbate 80; and optionally
- 1.0 wt.-% of a dispersing agent, preferably in the form of sorbitan sesquioleat.

In a further aspect, the present invention concerns methods for the production of oral thin films as noted above. In this respect, the oral thin film of the present invention can be prepared by any suitable technology or method, which is known and available to the person skilled in the art. Preferably, the oral thin film is produced by a method which involves the following steps:
a) producing a solution, dispersion or melt comprising the cariprazine or a pharmaceutically acceptable salt, tautomer or stereoisomer thereof and the water-soluble polymer, selected from pullulan and polyvinyl alcohol as film forming agent;
b) spreading the solution, dispersion or melt from step a);
c) drying the spread solution, dispersion or melt from step b) to obtain an oral thin film.

In a yet further aspect, the present invention relates to a therapeutic system in the form of an oral thin film as described in detail above, or an oral thin film, which is obtainable by the method as noted in the above aspect, for pharmaceutical purposes, namely for use as a medicament.

In a yet further aspect, the present invention relates to a therapeutic system in the form of an oral thin film as described in detail above, or an oral thin film, which is obtainable by the method as noted in the above aspect, for use in the treatment of mental diseases. In a yet further aspect, the present invention relates to the use of a therapeutic system in the form of an oral thin film as described in detail above for the manufacture of a medicament, particularly for the treatment of a mental disease. In a yet further aspect, thepresent invention relates to a method of treatment of a mental disease in a human patient comprising the administration of the oral thin film as defined above, in particular into the oral cavity of the patient.

For the oral thin film for use as medicament, the use of the oral thin film for the manufacture of a medicament, or the method of treatment as outlined above, it will be understood that caripzazine is administered by applying the oral thin film as defined above to the buccal, sublingual, gingival or palatal mucosa, more particularly, wherein said oral thin film provides a human patient in needs thereof with an amount of cariprazine which is effective in the treatment of a mental disease. As mental diseases, which can be treated with the respective films, schizophrenia, psychosis, bipolar disorder, bipolar depression, major depressive disorder and bipolar and acute mania can be mentioned. Patients with dysphagia are a particularly suitable patient group for such treatment.

The following section outlines particular embodiments of the present invention:
1. A therapeutic system in the form of an oral thin film for the administration of cariprazine, which comprises a matrix layer, and, wherein the matrix layer comprises:
   - cariprazine or a pharmaceutically acceptable salt, tautomer or stereoisomer thereof; and
   - film forming agent comprising a water-soluble polymer, which is selected from pullulan and polyvinyl alcohol,
      wherein pullulan and/or polyvinyl alcohol account for 57 wt.-% or more of the matrix layer.
2. Therapeutic system according to embodiment 1, where the cariprazine or a pharmaceutically acceptable salt, tautomer or stereoisomer thereof is in uncomplexed form.
3. Therapeutic system according to any of the preceding embodiments, wherein the film does not comprise hydroxypropylmethylcellulose and/or xanthan gum.
4. Therapeutic system according to any of the preceding embodiments, wherein cariprazine is present as powder having has a D90 particle size of > 30 µm, more preferably ≥ 40 µm and even more preferably ≥ 50 µm.
5. Therapeutic system according to any of the preceding embodiments, wherein cariprazine is present as powder having a D90 particle size of equal to or less than 100 µm, more preferably equal to or less than 90 µm and even more preferably equal to or less than 80 µm.
6. Therapeutic system according to any of the preceding embodiments, wherein wherein cariprazine is present as powder having has a D90 particle size of is in the range of from 50 to 80 µm.
7. Therapeutic system according to any of the preceding embodiments, wherein cariparzine is in the form of cariparzine hydrochloride, which is the form having market approval for therapy.
8. Therapeutic system according to any of the preceding embodiments, wherein the matrix layer comprises further water-soluble polymer.
9. Therapeutic system according to any of the preceding embodiments, wherein the combined amount of pullulan and/or polyvinyl alcohol on the total weight of water-soluble polymers in the matrix layer is at least 80 wt.-%, more preferably at least 90 wt.-% and even more preferably at least 95 wt.-%.
10.Therapeutic system according to any of the preceding embodiments, wherein the matrix layer is based on polyvinyl alcohol as the sole water-soluble polymer, more particularly, wherein said polyvinyl alcohol is characterized by one or more of the following parameters:
   i) degree of hydrolysis in the range from 80 % to 95% and more preferably 84 % to 92 %;
   ii) a viscosity as a 4% aqueous solution at 20°C according to DIN 53015 of 1 to 10 mPa.s and preferably 2 to 7 mPa.s.
11.Therapeutic system according to any of the preceding embodiments wherein
   the water-soluble polymer is present in an amount of 80 wt.-% or less, more preferably 75 wt.-% or less, even more preferably 70 wt.-% or less and even more preferably 65 wt.-% or less.
12.Therapeutic system according to any of the preceding embodiments, wherein the weight ratio, in which the cariprazine or a pharmaceutically acceptable salt, tautomer or stereoisomer thereof and polyvinyl alcohol are present therein is in the range of from 1:3 to 1:6 and preferably 1:3.5 to 1:5.
13.Therapeutic system according to any of the preceding embodiments which comprises additional auxiliary additives selected from plasticizers, humectants fatty acids, sweeteners, flavoring agents, colorants, taste masking agents, solubilizers, disintegrants, dispersing agents, emulsifiers, antioxidants, preservatives, stabilizers, and pH regulators.
14.Therapeutic system according to any of the preceding embodiments, wherein the combined amount of additives is in range of from 5 to 15 wt.-% and more preferably in the range from 8 to 14 wt.-%.
15.Therapeutic system according to any of the preceding embodiments, wherein the thickness of the film is 20 to 150 µm particularly preferably, the thickness of the film is in a range from 50 to 120 µm.
16.Therapeutic system according to any of the preceding embodiments, wherein the area weight of the film in the range from 20 to 180 g/m² preferably 50 to 140 g/m².
17.Therapeutic system according to any of the preceding embodiments, wherein the film has a size of 3 to 10 cm² and more preferably 4 to 6 cm².
18. Therapeutic system according to any of the preceding embodiments, wherein the content of cariprazine relative to the size of the film is in a range of from 0.3 to 3.0 mg/cm² and more preferably 0.5 to 2.0 mg/cm².
19. Therapeutic system according to any of the preceding embodiments, wherein the cariprazine or a pharmaceutically acceptable salt, tautomer or stereoisomer thereof is present in dispersed or in microparticulate form in the matrix layer.
20. Therapeutic system according to any of the preceding embodiments, which is a monolayer film.
21. Therapeutic system according to any of the preceding embodiments, which is characterized by a disintegration time in the oral cavity in the range of from 8 to 60 sec and more preferably of from 10 to 30 sec.
22. Therapeutic system according to any of the preceding embodiments, which comprises at least one of the following ingredients:
   - 2 to 20 wt.-%, preferably 5 to 15 wt.-% and particularly preferably about 14.5 wt.-% of cariprazine hydrochloride, preferably as uncomplexed cariprazine;
   - 57 to 65 wt.-% preferably 57 to 62 wt.-% of polyvinyl alcohol, preferably with a molecular weight Mw of 20,000 to 40,000 g/mol and in particular about 31,000 g/mol;
   - 9.0 wt.-% of a humectant, preferably in the form of sorbitol;
   - 3 to 9 wt.-% of a plasticizer, preferably in the form of glycerol;
   - 5.0 to 6.0 wt.-% of a disintegrant, preferably in the form of rice starch;
   - 3.0 wt.-% of sweetener, preferably selected form sucralose and xylitol;
   - 1.0 wt.-% of an emulsifier, preferably in the form of polysorbate 80; and optionally
   - 1.0 wt.-% of a dispersing agent, preferably in the form of sorbitan sesquioleat.
23. Method for the production of a therapeutic system according to any one of the preceding embodiments, comprising the following steps:
   a) producing a solution, dispersion or melt comprising the cariprazine or a pharmaceutically acceptable salt, tautomer or stereoisomer thereof and the water-soluble polymer, selected from pullulan and polyvinyl alcohol as film forming agent;
   b) coating the solution, dispersion or melt from step a);
   c) drying the spread solution, dispersion or melt from step b) to obtain an oral thin film.
24. A therapeutic system, which is obtainable by the method of embodiment 23.
25. The therapeutic system according to any of any of the embodiments 1 to 22 or 24, for use as medicament, particularly in the treatment of mental diseases.
26. The therapeutic system according to any of any of the embodiments 1 to 22 or 24, for use in the manufacture of a medicament, particularly for the treatment of a mental disease.
27.A method of treatment of a mental disease in a human patient comprising the administration of therapeutic system according to any of any of the embodiments 1 to 22 or 24, in particular into the oral cavity of the patient.
28. The therapeutic system of any of embodiments 25 to 26, or the method of embodiment 27, wherein the mental disease is selected from the group of schizophrenia, psychosis, bipolar disorder, bipolar depression, major depressive disorder, dysphagia, bipolar and acute mania.

In the following, the present invention will be further described by means of a number of examples, which however are not to be understood as limiting to the scope of the present invention.

### Examples

### Film preparation

Different compositions as shown in the below table 1 were formulated with cariprazine as the active ingredient. The formulations were prepared by first preparing an aqueous dispersion of cariprazine HCl, to which the water soluble polymers and other water soluble ingredients were added. The thus prepared mixture was then cast onto a polyethylene terephthalate release liner and dried to provide the respective films with an area weight of about 100 g/m² (exact area weights are given in the below table).

**Table 1**

| | Sample 1 | Sample 2 | Sample 3* | Sample 4 | Sample 5 |
|---|---|---|---|---|---|
| Cariprazine HCl | 14.5 % | 14.5 % | 14.5 % | 14.5 % | 14.5 % |
| PVA | 57.5 % | 61.5 % | | 58.5 % | |
| HPMC | | | 62.0 % | | |
| Pullulan | | | | | 60.5 % |
| Flavor | | | | | 4.0 % |
| Span 83 | 1.0 % | | | | 1.5 % |
| Xylitol | 2.0 % | 2.0 % | | 2.0 % | |
| Tween 80 | 1.0 % | 1.0 % | | 1.0 % | 1.5 % |
| Sorbitol | 9.0 % | 9.0 % | | 9.0 % | 9.0 % |
| Sucralose | 1.0 % | 1.0 % | 6.0 % | 1.0 % | 1.0 % |
| Glycerin | 9.0 % | 8.0 % | 15.0 % | 8.0 % | 3.0% |
| Rice starch | 5.0 % | 3.0 % | | 6.0 % | 5.0 % |
| Saccharin Na | | | 2.5 % | | |
| Area weight | 99.6 g/m² | 95.3 g/m² | 101.7 g/m² | 104.4 g/m² | 102.4 g/m² |
| API concentration | 1442.9 µg/cm² | 1375.4 µg/cm² | 1424.2 µg/cm² | 1510.9 µg/cm² | 1481.5 µg/cm² |

| | | | | | |
|---|---|---|---|---|---|
| * = not according to the invention | | | | | |

### Permeation study

The thus obtained films were tested within an in-vitro permeation test setup for uptake of the active ingredient with mucosa oesophagus skin (pig) with phosphate butter, pH 6.8 + 0.1 NaN₃ as acceptor medium. The film was applied to the dermatomized mucosa with 400 µm thickness and the mucosa with the film was immersed on the top side in the medium (the bottom side being in contact with acceptor medium with a temperature of 37°C, and the top side being compartmentalized to a mucosa area of 4.524 cm²). At predetermined time points, the acceptor medium used in each case was completely replaced by a new one and the content of permeated active ingredient in these acceptor solutions was determined by using HPLC.

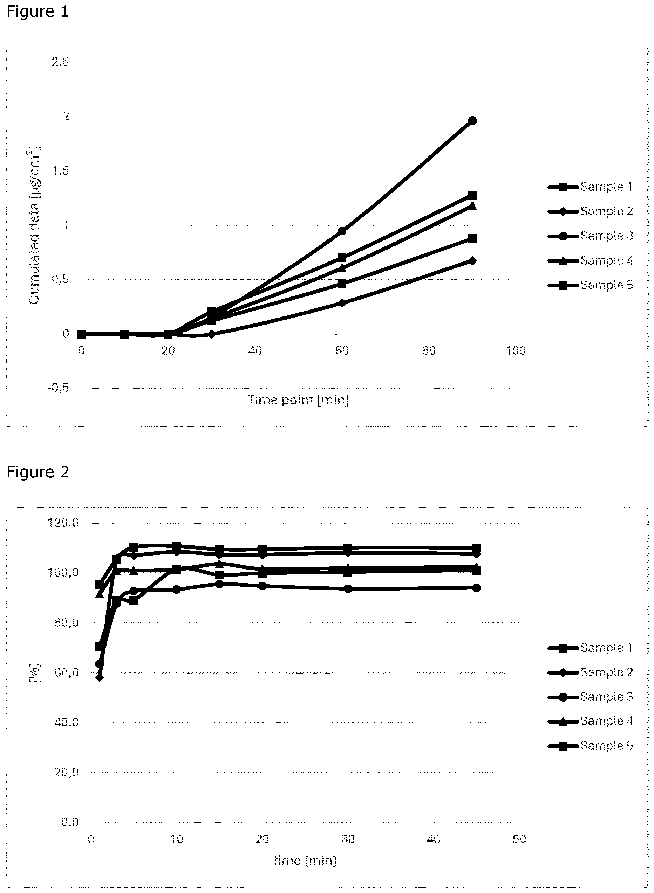

The results of these investigations are shown in Figure 1, where the respective sample numbers are given on the right hand side.

As is apparent from the graphs in Figure 1 at 30 Min some uptake of the cariprazine HCl was detected, which increased with increasing measuring time. In addition, it is apparent that the permeation was lower in the pullulan film sample and especially in the samples, which were formulated with PVA (polyvinyl alcohol) as the film forming agent. Accordingly, it is apparent that these films have less uptake of the active agent via the oral musosa which ensures that the predominant part of the active agent is swallowed and then taken up by the patient in the gastrointestinal tract.

### In-vitro dissolution study

The obtained films were also tested within an in-vitro dissolution test setup. In this trial the active ingredient is released from the oral thin film formulation and the active ingredient is determined by HPLC. The active ingredient is released in a sodium acetate buffer pH 5. The quantification was performed against an external standard.

The results of these investigations are shown in Figure 2.

As it is apparent from the graphs in Figure 2, PVA (polyvinyl alcohol) film samples show a higher and faster in-vitro release of the active ingredient than the pullulan and HPMC (hydroxypropyl methylcellulose) film samples.

## Claims

1. A therapeutic system in the form of an oral thin film for the administration of cariprazine comprising a matrix layer, wherein the matrix layer comprises:
- cariprazine or a pharmaceutically acceptable salt, tautomer or stereoisomer thereof; and
- film forming agent comprising a water-soluble polymer, selected from pullulan and polyvinyl alcohol,
wherein pullulan and/or polyvinyl alcohol account for 57 wt.-% or more of the matrix layer.

2. Therapeutic system according to claim 1 , where the cariprazine or a pharmaceutically acceptable salt, tautomer or stereoisomer thereof is in uncomplexed form.

3. Therapeutic system according to claim 1 or 2, wherein the film does not comprise hydroxypropylmethylcellulose and/or xanthan gum.

4. Therapeutic system according to any one of claims 1 to 3, wherein the film comprises 2 to 20 wt.-% and preferably 5 to 15 wt.-% of cariprazine or a pharmaceutically acceptable salt, tautomer or stereoisomer thereof, wherein the cariprazine preferably is cariprazine hydrochloride.

5. Therapeutic system according to any one of the preceding claims, wherein the water soluble polymer is polyvinyl alcohol, preferably wherein the polyvinyl alcohol has one or more of a molecular weight Mw in the range from 20,000 to 70,000 and preferably 25,000 to 35,000, a degree of hydrolysis in the range von 80 to 95 % and preferably 84 to 92 % and a viscosity as a 4% aqueous solution at 20°C according to DIN 53015 of 1 to 10 mPa.s and preferably 2 to 7 mPa.s.

6. Therapeutic system according to claim 5, wherein ratio of cariprazine or pharmaceutically acceptable salt, tautomer or stereoisomer thereof to polyvinyl alcohol by weight is in the range from 1:3 to 1:6 and preferably 1:3.5 to 1:5.

7. Therapeutic system according to any one of the preceding claims, wherein the matrix layer comprises the water-soluble polymer in an amount of from 57 to 80 wt.-%, preferably 57 to 75 wt.-%and more preferably 58 to 65 wt.-%.

8. Therapeutic system according to any one of the preceding claims, wherein the matrix layer further comprises one or more additives selected form the group comprising fatty acids, sweeteners, flavoring agents, colorants, taste masking agents, solubilizers, disintegrants, dispersing agents, emulsifiers, antioxidants, preservatives, stabilizers, and pH regulators, preferably wherein the additives account for 5 to 15 and preferably 8 to 14 wt.-% of the matrix layer.

9. Therapeutic system according to any one of the preceding claims, which has a film thickness in the range from 20 to 150 µm and preferably 50 to 120 µm and/or an area weight of 20 to 180 g/m² and preferably 50 to 140 g/m² and/or a size of 3 to 10 cm², preferably 4 to 6 cm², and/or which comprises the cariprazine in an amount from 0.3 to 3.0 mg/cm² and preferably 0.5 to 2.0 mg/cm².

10. Therapeutic system according to any one of the preceding claims, wherein the cariprazine or a pharmaceutically acceptable salt, tautomer or stereoisomer thereof is comprised in the matrix layer in dispersed or in microparticulate form.

11. Therapeutic system according to any one of the preceding claims, wherein the oral thin film has a dissolution/disintegration time in the oral cavity of from 8 to 60 sec and preferably 10 to 30 sec, and/or wherein the oral thin film is formulated as a flash release film.

12. Therapeutic system according to any one of the preceding claims, wherein the oral thin film comprises:
- 2 to 20 wt.-%, preferably 5 to 15 wt.-% and particularly preferably about 14.5 wt.-% of cariprazine hydrochloride, preferably as uncomplexed cariprazine;
- 50 to 65 wt.-% preferably 57 to 62 wt.-% of polyvinyl alcohol, preferably with a molecular weight Mw of 20,000 to 40,000 g/Moland in particular about 31,000 g/mol;
- 9.0 wt.-% of a humectant, preferably in the form of sorbitol;
- 3 to 9 wt.-% of a plasticizer, preferably in the form of glycerol;
- 5.0 to 6.0 wt.-% of a disintegrant, preferably in the form of rice starch;
- 3.0 wt.-% of sweetener, preferably selected form sucralose and xylitol;
- 1.0 wt.-% of an emulsifier, preferably in the form of polysorbate 80; and optionally
- 1.0 wt.-% of a dispersing agent, preferably in the form of sorbitan sesquioleat.

13. A method for the production of a therapeutic system according to any one of the preceding claims, comprising the following steps:
a) producing a solution, dispersion or melt comprising the cariprazine or a pharmaceutically acceptable salt, tautomer or stereoisomer thereof and the water-soluble polymer, selected from pullulan and polyvinyl alcohol as film forming agent;
b) spreading the solution, dispersion or melt from step a);
c) drying the spread solution, dispersion or melt from step b) to obtain an oral thin film.

14. Therapeutic system according to any one of claims 1 to 12 or obtainable by the method according to claim 13 for use as a medicament.

15. Therapeutic system according to any one of claims 1 to 12 or obtainable by the method according to claim 13 for use in the treatment of mental diseases, in particular schizophrenia, psychosis, bipolar disorder, bipolar depression, major depressive disorder and bipolar and acute mania, and preferably for the treatment of patients with dysphagia.
